# EUROPEAN PATENT SPECIFICATION

(11) **EP 1 137 381 B1**
(45) Date of publication and mention of the grant of the patent: **29.10.2003**
(21) Application number: 99965207.6
(22) Date of filing: 09.12.1999
(51) Int. Cl.: A61F 13/15, A61F 13/56

(54) **BREATHABLE ABSORBENT ARTICLES WITH PANTY FASTENING ADHESIVE AND ADHESIVE COMPONENT CONNECTIONS**
VERKLEBTER LUFTDURCHLÄSSIGER SAUGFÄHIGER GEGENSTAND MIT KLEBEBEFESTIGUNG AN DER UNTERWÄSCHE
ARTICLES ABSORBENT IMPER-RESPIRANTS DOTES D'ADHESIF DE FIXATION AUX SOUS-VETEMENTS ET DE LIAISONS ADHESIVES

(30) Priority: 10.12.1998 EP 98123642
(43) Date of publication of application: 04.10.2001
(73) Proprietor: The Procter & Gamble Company, Cincinnati, Ohio 45202 (US)
(72) Inventor: HIRSCH, Uwe, Thomas, Michael, Horst, D-64347 Griesheim (DE)
(74) Representative: Kremer, Véronique
(86) International application number: US9929331
(87) International publication number: WO00033781

(56) References cited:
- EP-A- 0 705 584
- EP-A- 0 745 368
- GB-A- 2 035 092

## Description

### Field of the invention

The present invention relates to an improvement for breathable absorbent articles such as sanitary napkins, catamenials or adult incontinence inserts which are used in combination with undergarments and known, for instance, from EP-A-0 705 584. The breathable absorbent articles according to the present invention are provided with a fastening adhesive to attach them to the undergarment during use. In particular the present invention relates to such absorbent articles which are made from several layers or components which are joined to each other along their periphery and held together across their surfaces by integrating adhesive connections. According to the present invention all of the integrating adhesive connections extend across a region which is co-extensive or less with the region occupied by the fastening adhesive.

### Background of the invention

Fastening adhesive for sanitary napkins are well-known in the art. Usually they cover part of the garment facing surface of sanitary napkins. The adhesive allows improved fixation of the napkins to the undergarment when applying the product and during use. One drawback of such adhesives for breathable articles which has been recognized is that the adhesive creates a barrier against water vapour transmission (occluding effect).

The dimensions of such panty fastening adhesives are influenced by several factors. One aspect is that they should cover as large an area as possible in order to provide firm attachment of the napkin to the undergarment. On the other hand the adhesive must not extend to areas which could possibly attach to the skin of the wearer since that would cause an unacceptable discomfort when using the product. Also the above mentioned occluding effect in the breathable article context has led to the need for limiting the extend of the panty fastening adhesive.

The other aspect when deciding on the panty fastening adhesive dimensions is how to provide the panty fastening adhesive onto the garment facing surface of a sanitary napkin in a mass production process. Many processes have been considered in this respect. EP-A-745 386 discloses printing of panty fastening adhesives in order to allow any shape of panty fastening adhesives to be provided. However, it is still most common to provide panty fastening adhesives by continuous coating or continuous spraying. The panty fastening adhesive is applied while the sanitary napkin moves on a band or belt in longitudinal direction. This automatically results in straight adhesive dimensions, which, when combined with an on-off switching of the adhesive application system, creates a rectangular stripe, or a series of rectangular stripes, on the garment facing surface of a sanitary napkin.

Therefore a compromise between the various needs and requirements of the dimension and design of the panty fastening adhesive has to be found. A common compromise are a series of 2 or 3 adhesive strips allowing breathability where the panty fastening adhesive is not and providing a large enough extend to ensure good holding of the article to the underlying garment.

However, once such a compromise is selected breathability will still suffer if internally other adhesive barriers block or occlude water vapour transmission. The adhesives used internally to hold the layers of the article together hence should be designed such that no additional occlusion occurs. This is achieved by the articles according to the present invention.

Of course the problem of occlusion is most prominent for long sanitary napkins, and in particular those which are asymmetrically shaped, i.e. having a longer and/or wider front or rear portion because these longer portions need a better attachment to the undergarment.

### Summary of the invention

The present invention relates to absorbent articles such as sanitary napkins for use in an undergarment. The absorbent article has a garment facing surface and a longitudinal centerline and a transverse centerline perpendicular to the longitudinal centerline which define a longitudinal direction and a transverse direction. The absorbent article is breathable and comprises at least 3 components. A generally liquid permeable topsheet, a generally liquid impermeable backsheet which allows water vapor transmission and an absorbent core between the topsheet and the backsheet. The absorbent article in each of its components have a garment facing surface and wearer facing surface. At least one of the wearer facing surfaces of the components is joined at least partially by a connecting adhesive to the garment facing surface of an immediately adjacent component. Thereby an integrating connection is formed which remains intact during use of the article. The integrating connection extends throughout a connection region.

On the garment facing side of the article a fastening adhesive for attachment of the article to the undergarment, also referred to as panty fastening adhesive, is provided. According to the present invention both types of adhesives, the one used for the panty fastening adhesive and the adhesives used for providing the integrating connections are occluding to water vapor transmission. According to the present invention each of the connection regions is either co-extensive with or it can be smaller than the region throughout which the panty fastening adhesive extends.

In a preferred embodiment all the components of the article are adhesively attached to each other by connecting adhesive. Further the article preferably has a length of at least 270 more preferably yet more than 300 mm in longitudinal direction.

In another alternative embodiment according to the present invention the article preferably has wings which can be either formed as integral extensions of at least the topsheet and the backsheet and are therefore (due to the materials) breathable or alternatively they can be joined to the article and be made from separate materials which also should be water vapor transmittened. As already indicated the preferred embodiment according to the present invention is a sanitary napkin or panty liner.

### Brief description of the drawings

Figure 1 shows a plan view of the garment facing surface of a sanitary napkin according to the present invention .

Figure 2 shows a preferred embodiment of a sanitary napkin according to the present invention with a fastening adhesive on the garment facing surface of the napkin.

### Detailed description of the invention

According to the present invention an absorbent article for use in combination with an undergarment has an adhesive for attachment of the article to the undergarment. The design of the adhesive is particularly adjusted to the dimensions of the article and can preferably be asymmetric in longitudinal direction and in use reaches into a region in the undergarment distant from the crotch center. The layered components of the articles are attached to each other to integrate them during use.

In particular, sanitary napkins, catamenials and panty liners whether used for incontinence discharges or menstrual or other discharges are considered to be susceptible to the present invention. Typically such articles are of layered construction with each layer or group of layers and the article having a garment facing surface which is oriented to face in the direction of a garment during use of the article and a wearer facing surface facing in the opposite direction. Typically such articles comprise a liquid pervious topsheet forming the wearer facing surface of the article, an absorbent core and a breathable backsheet forming the garment facing surface of the article. The absorbent core is interposed between the topsheet and the backsheet. All components of the article are encased by the topsheet and the backsheet which are hence preferably joined along their peripheral edge to each other. All other components also extending to the peripheral edge of the article can, and preferably are, also joined to the peripheral edge of the topsheet and/or backsheet. These peripheral joinders are in addition and not replacing the adhesive attachments within the article.

### Absorbent Article Components

### The topsheet

In general, the topsheet should have good liquid retention to maintain a dry surface and thereby keep the skin of the wearer dry; the absorbent core needs to provide enough absorbent capacity and the backsheet should prevent wet through (liquid permeability) to retain the absorbed fluid while preferably being breathable.

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are typically selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the wearer remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,391. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways are also contemplated by the present invention such as for example only a portion of the topsheet comprising liquid passage ways or a film with apertures of various sizes. Typically such topsheets would have the liquid passage ways oriented such that they result in a centrally permeable and peripherally impermeable topsheet for liquids.

The wearer facing surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the wearer facing surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure.

When referring to the topsheet a multi layer structure or a mono layer structure is contemplated. The hybrid topsheet mentioned above is such a multi layer design but other multi layer topsheets such as primary and secondary topsheet designs are also considered.

### The core

Positioned in fluid communication with, and typically underlying the topsheet is the absorbent core. The absorbent core provides fluid storage and distribution function and can also comprise multiple layers. The core can comprise any usual absorbent material or combinations thereof. It preferably comprises absorbent gelling materials usually referred to as "hydrogel", "superabsorbent", hydrocolloid" materials in combination with suitable carriers.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers, such as acrylic acid, which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of this article.

An embodiment of the core, particularly useful in the application of the present invention, comprises a double layer tissue laminate which can be formed by folding the tissue onto itself. These layers can be joined to each other. Absorbent gelling material or other optional material can be comprised between the layers.

The absorbent core can include optional components normally present in absorbent webs such as odor control agents, in particular suitable zeolites or silicas.

### The backsheet

The backsheet primarily prevents the exudates absorbed and contained in the absorbent core from wetting articles that contact the absorbent product such as underpants, pants, pyjamas and undergarments. The backsheet is preferably impervious to liquids (e.g. menses and/or urine) and usually manufactured from a thin plastic film.

The backsheet typically extends across the whole of the absorbent core and can extend onto and form part of the topsheet by folding around the absorbent core. Thereby a topsheet configuration as disclosed in US 4,342,314, column 16, lines 47 - 62 can be achieved without the requirement to selectively aperture the topsheet.

The backsheet also provides breathability to the absorbent article by being at least water vapour permeable, preferably air permeable. The backsheet can be a laminate material e.g. of a combination of microporous film and/or non-woven material, and/or apertured formed film. The layers can be joined to each other by an adhesive. Breathability if desired can be limited to the periphery or the center of the backsheet or it can be across the whole backsheet.

### The panty fastening adhesive

The sanitary napkin comprises an adhesive area on its garment facing surface which adhesive is protected by a cover means which cover means is released prior to use of the article. The adhesive area need not be fully covered by adhesive but may for example be provided by sub portions of adhesive and is then defined as the area formed by the shortest possible line encircling all sub portions, however excluding those in the wings.

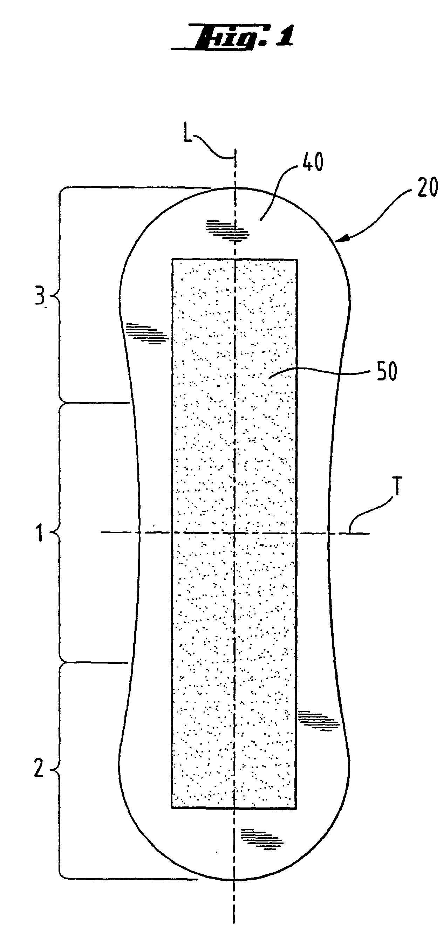

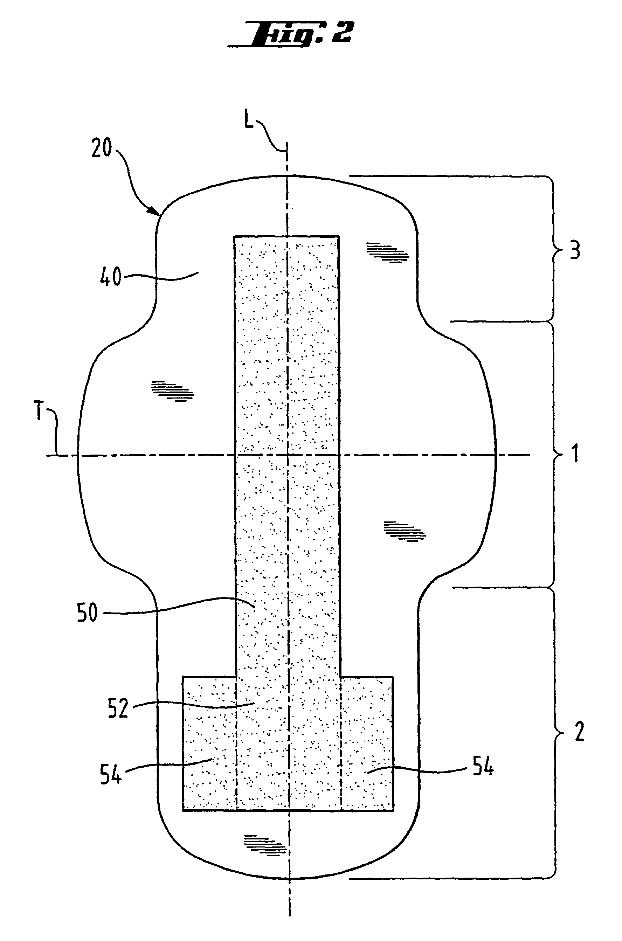

As can be seen in the sanitary napkin (20) of figure 1 the garment facing side (40) which typically is provided by a breathable but liquid impermeable backsheet of such a sanitary napkin, comprises an adhesive (50). In general sanitary napkins have a longitudinal centerline (L) and a transverse centerline (T). In the sanitary napkin of Figure 1 the transverse centerline (T) is located in the middle of the sanitary napkin, i.e. it separates the sanitary napkin into two halves which are approximately of equal length in longitudinal direction. The shown sanitary napkin is also symmetrical in longitudinal direction to the transverse centerline (T). In contrast the sanitary napkins shown in figure 2 has a transverse centerline (T) which separates a napkin into two halves which are substantially not identical in length.

The transverse centerline (T) is located in a central or first region of a sanitary napkin. This first region has an extension in longitudinal direction sufficient and intended to cover the area on a wearer between the perineum and the most forward point of the labia majora. Based on medical studies this varies between about 40 mm and 80 mm on average women depending on sexual activity, child birth and other factors. Hence the minimum length of the first region is 40 mm but would preferably be at least 60 mm, more preferably at least 80 mm and most preferably at least 112 mm. The transverse centerline separates the first region into two halves in longitudinal direction which are substantially of equal length. This is the case for symmetrical sanitary napkins or for asymmetrical sanitary napkins.

Sanitary napkins having a first region (1) according to the above definition also have a second (2) and a third (3) region outside the first region which are extending from the first region (1) to the longitudinal ends of the sanitary napkin. As can be seen from figure 2 a sanitary napkin can have a second region (2) which is longer than the third region (3).

The panty fastening adhesive (50) in figure 1 extends an equal distance from the first region (1) into the second (2) and the third region (3). In contrast asymmetric sanitary napkins have an undergarment adhesive attachment (50) which preferably extends in longitudinal direction further from the first region (1) into the second region (2) than it extends from the first region into the third region (3), as shown in the figure 2.

According to the present invention the width in transverse direction of the adhesive in the second region (2) can be wider in at least one point than the width of the adhesive in the first (1) and in the third region (3). Figure 2 shows a particularly preferred shape of adhesive in which the adhesive is provided in a rectangular pattern, which is easy to provide when continuously manufacturing sanitary napkins in longitudinal direction.

It is also possible that the adhesive (50) in the wider portion of the adhesive in the second region (2) of sanitary napkins according to the present invention may comprise a different quantity of adhesive than the adhesive in the first or second region. For example the pattern shown in figure 2 can be provided by coating an adhesive in longitudinal direction through three coating means (e.g. slot coaters or spray nozzles). One coating means providing a central rectangular area (54) extending from the third (3) through the first (1) to the second (2) region (indicated by dashes in the second region (2)) and being similar to the adhesive shown in figure 1. In addition the areas of adhesive (54) extending beyond the rectangular portion (52) in the second region (2) of the sanitary napkin are provided by two additional coating means which may provide the same amount of adhesive per area or may provide a different amount of adhesive per area than the central coating means. In principle it would also be possible to provide different adhesives through the different coating means. In this context also stripes of adhesive instead of a completely coated surface could be provided.

In an alternative way of providing the adhesive to the garment facing surface (40) of a sanitary napkin according to the present invention it would also be possible to provide the whole area in which the adhesive is wider in the second region (2) by a separate coating means while the adhesive in the first and third region of the sanitary napkin is provided by conventional adhesive coating means such as those used for prior art sanitary napkins according to figure 1.

As noted above sanitary napkins according to the present invention are preferably provided with flaps or wings extending beyond the undergarment crotch width and being folded around the undergarment crotch so as to protect the undergarment side edges during use. Sanitary napkins with wings are shown in the preferred embodiment of figure 2.

The adhesive for fastening the article in an undergarment can be any adhesive useful for the desired application. Usually it is a pressure sensitive adhesive which can be applied in any way usual in the art. In particularly, the adhesive application can be by use of contact application means, such as printing or scraping or slot coating, or by non contact application, such as curtain coating, spraying or spiraling. The adhesive can be applied as hot melt or from a cold solution, either to the garment facing surface of the article or to the cover means which then carries it to the article. According to the present invention the adhesive has an adverse effect on breathability. Where the adhesive is coated onto the backsheet water vapor permeation or air permeation is reduced or even blocked. This is referred to as occluding adhesive.

In general the adhesive should be adapted to the desired application. The adhesion force of the adhesive to the undergarment facing surface of the article needs to be larger or equal to that adhesion force which it has to the cover means. Also it is desirable to have an adhesive which does not separate during use from the article and leaves adhesive traces in the undergarment when separating the article from the undergarment. Finally the adhesive (and hence the adhesive area) need to provide firm attachment to the undergarment during use.

### Joining the components of the article together

According to the present invention the absorbent article components are joined to each other in an integrating fashion. This is usually needed to ensure that the forces in a direction perpendicular to the layered components can be transmitted between the layers of the components such that all components of the article are held to the undergarment in a controlled fashion through the panty fastening adhesive during the whole usage period of the article. This joining of the components is necessarily provided (and needed) most in the central area of the article. In addition it is usual to provide a peripheral joining of all components extending into the periphery of the article as indicated above. Most commonly these are the topsheet and the backsheet and their respective layers.

In order to achieve the integration of the article inside the peripheral rim joinder the wearer facing surface and the garment facing surface of the layers of the components are joined to each other by an adhesive connection. The connecting adhesive can be any which is usual in the art and its application can also be any conventional application. The preferred applications are the same as mentioned above for the panty fastening adhesive.

One requirement for the adhesive to be used for the integrating connections is that it remains intact during usual use of the absorbent article. Therefore it must not dissolve in the liquid absorbed by the absorbent article. In addition the present invention of course only applies to such adhesives which within the absorbent article cause an occlusion to water vapor transmission. If the adhesive is not occluding water vapor transmission or the adhesive is only provided in a non occluding fashion then the resulting connection does not need to satisfy the requirements of the present invention.

The integrating connection extends throughout a connection region. The connection region does not have to extend across the whole surface area of the article but in order to optimize its integrating function it preferably extends throughout about the same area as the panty fastening adhesive.

According to the present invention integrating connections between layers do not increase the area of occlution in a breathable absorbent articles. This is achieved when providing an adhesive connection between layers of the components of the article which extends in a connection region which is co-extensive with or smaller than the region throughout which the panty fastening adhesive extends.

The benefit of the breathability supporting integrating connection is only achieved when providing all of the required attachments between layers of the components of the article with such integrating connections or in a non-occluding way.

An additional benefit of providing integrating connections particularly for thin, highly flexible and drapable (schmiegsam) articles other than control of the components of the article in respect to the undergarment during use is the handling of the absorbent article when manipulating it from the condition in which it is provided to the wearer into the condition in which it is used. In other words thin, highly flexible and drapalbe (schmiegsam) articles without the integrating connections according to the present invention have a higher probability of being improperly handled such as for example crumpled or deformed already upon placement in the undergarment.

It will be appreciated by those skilled in the art that the above explanations and embodiments are disclosed for the purpose of illustration and enablement but that the present invention is defined by the limits given in the claims.

## Claims

1. A breathable disposable absorbent article (20) for use in an undergarment comprising at least three components
- a generally liquid permeable topsheet,
- a generally liquid impermeable backsheet, which allows water vapour transmission,
- an absorbent core between said topsheet and said backsheet,
- said article (20) having a longitudinal centerline (L) and a transverse centerline (T) perpendicular to said longitudinal centerline (L), said article and each of said components having a garment facing surface and a wearer facing surface, at least one of said wearer facing surfaces of said components being joined at least partially by a connecting adhesive to said garment facing surface of an immediately adjacent component of said article (20) thereby forming an integrating connection between said surfaces, said integrating connection remaining intact during usual use of said article (20), and said integrating connection extends throughout a connection region, said garment facing side (40) of said article (20) comprises a fastening adhesive (50) for attachment of said article (20) to said undergarment, said fastening adhesive and said connecting adhesive are occluding water vapour transmission, said article being **characterized in that**
- each of said connection regions is co-extensive with or smaller than the region throughout which said fastening adhesive (50) extends.

2. An article (20) according to claim 1 **characterized in that** all components of said article are provided with integrating connections.

3. An article (20) according to any of the preceding claims **characterized in that** said article (20) has a length of at least 270 mm, preferably at least 300 mm.

4. An article (20) according to any of the preceding claims **characterized in that** said article (20) further comprises wings (25) which are either
- formed as integral extensions of at least said topsheet and said backsheet, or alternatively
- joined to said article (20) and which are water vapour transmitting.

5. An article (20) according to any of the preceding claims which is a sanitary napkin or panty liner.

## Patentansprüche

1. Atmungsfähiger absorbierender Wegwerfartikel (20) zur Verwendung in einer Unterbekleidung umfassend mindestens drei Bestandteile
- eine im Wesentlichen flüssigkeitsdurchlässige Decklage,
- eine im Wesentlichen flüssigkeitsundurchlässige Außenlage, welche einen Wasserdampf-Durchgang gestattet,
- einen absorbierenden Kern zwischen der Decklage und der Außenlage,
- wobei der Artikel (20) eine Längs-Mittellinie (L) und eine zu der Längs-Mittellinie (L) senkrechte Quer-Mittellinie (T) aufweist, wobei der Artikel und jeder der Bestandteile eine Kleidungsstückzugewandte Oberfläche und eine Träger-zugewandte Oberfläche aufweisen, wobei mindestens eine der Träger-zugewandten Oberflächen der Bestandteile durch einen Verbindungsklebstoff mindestens teilweise mit der Kleidungsstück-zugewandten Oberfläche eines unmittelbar benachbarten Bestandteils des Artikels (20) verbunden ist, wodurch eine zusammenfügende Verbindung zwischen den Oberflächen gebildet wird, wobei die zusammenfügende Verbindung während des gewöhnlichen Gebrauchs des Artikels (20) intakt bleibt, und wobei die zusammenfügende Verbindung über einen gesamten Verbindungsbereich verläuft, wobei die Kleidungsstück-zugewandte Seite (40) des Artikels (20) einen Befestigungsklebstoff (50) zum Befestigen des Artikels (20) an der Unterbekleidung aufweist, wobei der Befestigungsklebstoff und der Verbindungsklebstoff einen Wasserdampf-Durchgang sperren, wobei der Artikel **dadurch gekennzeichnet ist, dass**
- jeder der Verbindungsbereiche eine gleiche Ausdehnung wie der Bereich oder eine kleinere als dieser aufweisen, über welchen sich der Befestigungsklebstoff (50) erstreckt.

2. Artikel (20) nach Anspruch 1, **dadurch gekennzeichnet, dass** alle Bestandteile des Artikels mit zusammenfügenden Verbindungen versehen sind.

3. Artikel (20) nach einem der vorherigen Ansprüche, **dadurch gekennzeichnet, dass** der Artikel (20) eine Länge von mindestens 270 mm, vorzugsweise von mindestens 300 mm aufweist.

4. Artikel (20) nach einem der vorherigen Ansprüche, **dadurch gekennzeichnet, dass** der Artikel (20) ferner Flügel (25) aufweist, die entweder
- als integrale Verlängerungen von mindestens der Decklage und der Außenlage ausgebildet sind, oder alternativ
- mit dem Artikel (20) verbunden sind und welche Wasserdampfdurchlässig sind.

5. Artikel (20) nach einem der vorherigen Ansprüche, welcher eine Damenbinde oder eine Slipeinlage ist.

## Revendications

1. Article absorbant jetable (20), apte à respirer, destiné à être utilisé dans un sous-vêtement, comprenant au moins trois composants :
- une feuille de dessus généralement perméable aux liquides ;
- une feuille de fond généralement imperméable aux liquides, qui permet la transmission de la vapeur d'eau ; et
- une âme absorbante entre ladite feuille de dessus et ladite feuille de fond ;
ledit article (20) ayant une ligne médiane longitudinale (L) et une ligne médiane transversale (T) perpendiculaire à ladite ligne médiane longitudinale (L) ; ledit article et chacun desdits composants ayant une surface côté vêtement et une surface côté utilisateur ; au moins l'une desdites surfaces côté utilisateur desdits composants étant au moins partiellement réunie, par un adhésif de liaison, à ladite surface côté vêtement d'un composant immédiatement adjacent dudit article (20), ce qui forme une liaison d'unification entre lesdites surfaces, ladite liaison d'unification restant intacte pendant une utilisation normale dudit article (20) et s'étendant sur toute une région de liaison ; ladite face côté vêtement (40) dudit article (20) comprenant un adhésif de fixation (50) pour la fixation dudit article (20) audit sous-vêtement ; ledit adhésif de fixation et ledit adhésif de liaison empêchant la transmission de la vapeur d'eau ; ledit article étant **caractérisé en ce que** :
chacune desdites régions de liaison est de même étendue ou est plus petite que la région dans laquelle s'étend ledit adhésif de fixation (50).

2. Article (20) selon la revendication 1, **caractérisé en ce que** tous les composants dudit article sont pourvus de liaisons d'unification.

3. Article (20) selon l'une quelconque des revendications précédentes, **caractérisé en ce que** ledit article (20) a une longueur d'au moins 270 mm, de préférence d'au moins 300 mm.

4. Article (20) selon l'une quelconque des revendications précédentes, **caractérisé en ce que** ledit article (20) comprend, en outre, des ailes (25) qui sont :
- réalisées sous forme d'extensions solidaires d'au moins ladite feuille de dessus et ladite feuille de fond, ou en variante
- réunies audit article (20),
et qui permettent la transmission de la vapeur d'eau.

5. Article (20) selon l'une quelconque des revendications précédentes, qui est une serviette hygiénique ou un protège-slip.
